Europäisches Patentamt

European Patent Office

Office européen des brevets

(11) Numéro de publication: **0 343 064**
**A1**

## (12) DEMANDE DE BREVET EUROPEEN

(21) Numéro de dépôt: **89401357.2**

(22) Date de dépôt: **17.05.89**

(51) Int. Cl.⁴: **G 01 N 33/68**
**G 01 N 33/02**

(30) Priorité: **19.05.88 FR 8806707**

(43) Date de publication de la demande:
**23.11.89 Bulletin 89/47**

(84) Etats contractants désignés:
**AT BE CH DE ES FR GB GR IT LI LU NL SE**

(71) Demandeur: **INSTITUT NATIONAL DE LA RECHERCHE AGRONOMIQUE (INRA)**
**147, Rue de l'Université**
**F-75007 Paris Cédex 07 (FR)**

(72) Inventeur: **Paraf, Alain**
**Maison Vert Cellettes**
**F-41120 Les Montils (FR)**

**Breton, Christel**
**11, Rue du Doussais**
**F-44880 Sautron (FR)**

**Aynaud, Jean-Marie**
**20, Rue Eupatoria**
**F-37000 Tours (FR)**

(74) Mandataire: **Ores, Irène et al**
**CABINET ORES 6, Avenue de Messine**
**F-75008 Paris (FR)**

(54) **Réactifs et procédé de dosage immunochimique du degré de dénaturation de substances protéiques.**

㊗ Réactif immunochimique d'identification et de quantification d'au moins une substance protéique et de la reconnaissance de la nature et du degré du traitement dénaturant auquel elle est soumise.

Ce réactif est constitué par des anticorps, polyclonaux ou monoclonaux, dirigés contre ladite substance protéique et présentant différentes spécificités qui correspondent chacune à un état de la substance protéique. Procédé de production d'un tel réactif.

Application à l'identification et à la quantification d'une substance protéique, notamment alimentaire et à la reconnaissance de la nature et du degré du traitement dénaturant appliqué à cette substance.

**Description**

## REACTIFS ET PROCEDE DE DOSAGE IMMUNOCHIMIQUE DU DEGRE DE DENATURATION DE SUBSTANCES PROTEIQUES

La présente invention est relative à des réactifs et à un procédé de dosage immunochimique du degré de dénaturation de substances protéiques, telles notamment que celles contenues dans des produits alimentaires soumis à des traitements de dénaturation thermiques, physiques, chimiques ou enzymatiques.

Il est important de pouvoir contrôler les procédés utilisés dans les industries agro-alimentaires :

1. du point de vue hygiénique pour identifier les agents pathogènes ou toxiques susceptibles d'être présents dans l'aliment ;

2. du point de vue technologique pour s'assurer que le produit a subi le procédé industriel dans son intégralité : par exemple, il est nécessaire de s'assurer qu'une viande dans une conserve ou dans un aliment préparé a été chauffée à 65°C (norme USDA) pour être certain que le produit ne renferme pas de germes pathogènes tels que colibacilles, salmonella ou staphylocoques.

3. du point de vue commercial, il est nécessaire de s'assurer que le produit ne contienne pas d'additifs frauduleux susceptibles d'amener à une altération de l'aliment (tels que nitrites ou nitrates) ou une modification organoleptique.

4. du point de vue des procédés utilisés, afin que l'on puisse s'assurer du bon développement des techniques utilisées, qu'il s'agisse de fermentation, de chauffage, d'additifs, d'extraction etc...

Différentes études ont été réalisées pour rechercher des relations entre structures physico-chimiques, en particulier des protéines, structures antigéniques et fonctions des protéines. On a également recherché de telles corrélations pour détecter la dénaturation par exemple d'une enzyme ou les modifications apportées à une structure par la dénaturation ou par les enzymes hydrolytiques.

L'analyse des structures moléculaires, en particulier des protéines, a pu être réalisée avec des substances pures, cristallisées ou en solution : des méthodes physiques, chimiques ou immunochimiques d'études ont été mises au point : pour ces dernières les anticorps polyclonaux ou monoclonaux ont la propriété de se fixer sur des molécules natives solubles, ce qui permet la reconnaissance de sites antigéniques appelés épitopes.

La littérature en la matière décrit en outre des travaux montrant qu'il est possible de détecter et d'identifier par des anticorps polyclonaux ou monoclonaux, des substances dans des mélanges complexes n'ayant subi aucun traitement.

Depuis quelques années, l'on s'est attaché à identifier des substances dans des mélanges complexes, même après chauffage. Notamment, on a pu montrer qu'il existait dans les muscles de différentes espèces, des protéines thermorésistantes qui pouvaient être extraites et reconnues par leur activité enzymatique spécifique. En utilisant la lactate déshydrogénase comme protéine-test on a pu montrer que le chauffage de la viande à 65°C diminue l'activité de cette enzyme (Keeton J.T. Collins S.S. et Smith S.B.- 34th European meeting of Meat Research Workers 1987 Helsinki) mais la technique n'est pas assez précise pour assurer que l'ensemble a été chauffé à au moins 65°C et le test n'est pas applicable aux températures supérieures. L'on peut, avec des anticorps spécifiques, identifier l'origine de la viande qui a été autoclavée (Erastus K. Kangethe and Kaare J. Lindqvist J. Sci. Food Agric. 1987, 179).

De la même manière, des anticorps dirigés contre la protéine du soja ont permis, par une technique immunochimique, de reconnaître la présence de soja dans des mélanges de viandes, de façon qualitative après chauffage à 100°C pendant cinq minutes (Frederik W. Janssen, Gerritt Voortman and Johannes A. de Baaii, Lebensm, Untersforsch, 1986, 182, 479).

En ce qui concerne l'ovalbumine purifiée on a pu reconnaitre les altérations immunochimiques produites par la glycosylation de la molécule au cours de la réaction de Maillard réalisée à 50°C, mais aucune application n'en a été faite sur des mélanges d'aliments (Yasuko Kato, Tsukasa Matsuda, Kenji Watanabe et Ryo Nakamura, Agric. Biol. Chem. 1985, 49, 423).

Il a été également montré que l'on peut obtenir des anticorps monoclonaux contre l'ovalbumine, qui différencient l'ovalbumine native de l'ovalbumine dénaturée par l'urée. Certains de ces anticorps monoclonaux sont également capables de reconnaître l'ovalbumine pure chauffée à 75°C, mais ici encore l'utilisation de tels anticorps n'a été réalisée ni pour des milieux complexes, ni pour identifier les différentes étapes de la dénaturation de l'ovalbumine (Kilshow et al. Clin. Exp. Immunol. 1986, 66, 481).

Une technique de détection d'ovalbumine dénaturée et d'ovalbumine native par des anticorps polyclonaux purifiés par chromatographie d'affinité, dirigés contre l'ovalbumine native, en utilisant la méthode ELISA, a été décrite dans J. OF FOOD SCI. Vol. 52, N°5, 1987, C. BRETON, L. PHAN THANH et A. PARAF "Immunochemical Properties of native and Heat denatured ovalbumin" et l'application de cette méthode à l'identification et à la quantification d'ovalbumine utilisée comme additif dans des champignons en boîte, a été décrite dans un autre Article de la même publication, par les mêmes Auteurs.

Il résulte de ce qui précède qu'aucune méthode immunochimique n'a été trouvée qui permette à la fois :

1. d'identifier une substance (en particulier protéique)

2. de quantifier une substance (en particulier protéique)

3. de définir à quelle température le produit destiné à l'alimentation a été chauffé ou quel traitement dénaturant physique, chimique ou enzymatique a été appliqué et à quel degré.

La présente invention s'est en conséquence donné pour but de pourvoir à des réactifs immunochimiques qui permettent à la fois d'identifier une substance protéique, de la quantifier et de définir la nature du traitement dénaturant qu'elle a subi et à quel degré elle a subi ce traitement dénaturant.

La présente invention s'est encore donné pour but de pourvoir à des réactifs immunochimiques très sensibles, aptes à analyser et à quantifier rapidement une ou plusieurs protéines contenues dans un produit alimentaire, lesquels réactifs sont stables, d'un prix de revient économiquement acceptable, disponibles en quantités importantes et permettent la réalisation des analyses susdites en grandes séries avant, durant, et/ou après le traitement auquel sont soumis les produits alimentaires analysés, permettant de suivre les conséquences dudit traitement sur ledit produit alimentaire.

La présente invention a pour objet un réactif immunochimique d'identification et de quantification d'au moins une substance protéique et de reconnaissance de la nature et du degré du traitement dénaturant auquel elle est soumise, lequel réactif est caractérisé en ce qu'il est constitué par des anticorps dirigés contre ladite ou lesdites substance(s) protéique(s) et présentant différentes spécificités qui correspondent chacune à un état de ladite ou desdites substance(s) protéique(s).

Selon un mode de réalisation avantageux du réactif immunochimique conforme à l'invention, celui-ci est constitué par des anticorps dirigés contre ladite (lesdites) substance(s) protéique(s) à l'état natif et/ou contre ladite (lesdites) substance(s) protéique(s) dénaturée(s) à différentes températures et/ou contre ladite (lesdites) substance(s) protéique(s) dénaturée(s) par un trai tement approprié tel, notamment, qu'un traitement physique, un traitement chimique ou un traitement enzymatique.

Selon un autre mode de réalisation avantageux du réactif immunochimique conforme à l'invention, celui-ci est dirigé contre au moins une substance protéique qui fait -ou font- partie des constituants du produit alimentaire analysé.

Selon encore un autre mode de réalisation avantageux du réactif immunochimique conforme à l'invention, celui-ci est dirigé contre au moins une substance protéique-test ajoutée au produit alimentaire à analyser, préalablement au traitement thermique, physique, chimique, enzymatique ou autre, auquel elle doit être soumise.

Selon un autre mode de réalisation avantageux du réactif immunochimique conforme à l'invention, celui-ci est constitué par des anticorps polyclonaux.

Selon encore un autre mode de réalisation avantageux du réactif immunochimique conforme à l'invention, celui-ci est constitué par des anticorps monoclonaux.

La présente invention a également pour objet un procédé de production du réactif immunochimique conforme à l'invention, qui est caractérisé en ce que des mammifères ou des oiseaux appropriés sont immunisés par au moins une substance protéique présentant différents états, et notamment différents états de dénaturation, en ce que les anticorps polyclonaux ou monoclonaux obtenus à la suite de cette immunisation et dirigés contre les différents états de ladite substance protéique, sont recueillis pour servir de reactifs immunochimiques aptes à détecter et à quantifier les différents états de dénaturation de ladite substance dans un produit alimentaire.

Selon un mode de mise en oeuvre du procédé de production du réactif conforme à l'invention, la substance protéique utilisée dans plusieurs de ses états, notamment de dénaturation, pour immuniser des mammifères ou des oiseaux appropriés, est identique à une substance protéique qui entre dans la composition du produit alimentaire à analyser.

Selon un autre mode de mise en oeuvre du procédé de production du réactif, conforme à l'invention, la substance protéique utilisée dans plusieurs de ses états, notamment de dénaturation, pour immuniser des mammifères ou des oiseaux appropriés, est une substance protéique identique à une protéine ajoutée au produit alimentaire à analyser, en tant que protéine-test connue pour son aptitude à entrainer l'apparition de différents déterminants antigéniques sensibles aux anticorps susdits.

Selon un autre mode de mise en oeuvre du procédé conforme à l'invention, appliqué à la production d'anticorps polyclonaux, le(s) serum(s) recueilli(s) après immunisation de mammifères ou d'oiseaux appropriés par au moins une protéine-constituant du produit alimentaire ou par ou moins une protéine-test native ou dans différents états de dénaturation, et contenant des anticorps polyclonaux est/sont traité(s) par un agent de précipitation propre à provoquer la précipitation des immunoglobulines pour éliminer des protéines sériques susceptibles de diminuer la sensibilité de la méthode.

Selon un mode de mise en oeuvre du procédé conforme à l'invention, appliqué à la production d'anticorps polyclonaux, le(s) serum(s) recueilli(s) après immunisation de mammifères ou d'oiseaux appropriés par au moins une protéine-constituant du produit alimentaire ou par au moins une protéine-test, dans différents états de dénaturation, et contenant des anticorps polyclonaux, est/sont traité(s) sur une colonne de chromatographie d'affinité pour séparer les différentes spécificités contenues dans lesdits sérums anti-substance protéique en vue de leur utilisation comme réactifs aptes à reconnaître chacune l'un des différents états de ladite substance protéique, dans le produit alimentaire à analyser.

La présente invention a en outre pour objet un procédé d'identification et de quantification d'au moins une substance protéique et de reconnaissance de la nature et du degré du traitement dénaturant auquel elle est soumise, caractérisé en ce qu'un réactif immunochimique, tel qu'un anticorps, apte à détecter différents états , notamment de dénaturation, d'une substance protéique présente dans un produit à analyser, notamment dans un produit alimentaire, est mis en contact avec le produit à analyser, puis le complexe antigène-anticorps formé est détecté et quantifié par une technique immunoenzymatique appropriée.

Selon un mode de mise en oeuvre préféré du procédé d'identification, quantification, reconnaissance,

3

conforme à l'invention, le produit à analyser est soumis préalablement à la détection de l'antigène (protéique) qu'il contient par une technique immunoenzymatique, à un processus d'extraction propre à solubiliser les protéines présentes dans ledit produit.

Les réactifs de dosage conformes à la présente invention permettent de caractériser le degré de dénaturation des produits alimentaires et en particulier permettent de déterminer la température à laquelle les produits ont été soumis.

Ces réactifs sont constitués par des anticorps polyclonaux ou monoclonaux : chaque série d'anticorps polyclonaux ou monoclonaux dirigés contre une protéine déterminée permet d'identifier ou bien la molécule de protéine native ou bien la molécule de protéine dénaturée ou des structures intermédiaires ; de plus, la méthode utilisée permet de mesurer de façon quantitative la molécule analysée. Les anticorps sont préparés contre une ou plusieurs protéines à analyser et sont utilisés sur des produits finis tels que pâtes, plats cuisinés, viandes et autres produits carnés, notamment pâtés, sur les poissons, sur les oeufs, le lait, les produits laitiers (tels que fromages, yaourts, crèmes desserts, etc...), sur les produits végé taux faits à partir de blé, de riz, de mais, de soja, par exemple, etc...

Conformément à l'invention, celle-ci comprend également l'introduction, préalablement au traitement du produit alimentaire, d'une protéine-test étrangère à l'aliment (par exemple ovalbumine, serumalbumine bovine, inhibiteurs de trypsine, caséines, β-lactoglobuline, etc...) : les réactifs de dosage conformes à l'invention, constitués par une série d'anticorps polyclonaux ou monoclonaux dirigés contre une protéine, telle que l'ovalbumine par exemple, permettent de détecter et de quantifier ladite protéine et de déterminer la température à laquelle l'aliment a été chauffé. L'identification des anticorps reconnaissant les différentes structures peut être réalisée par la technique ELISA.

Il est ainsi possible à la fois d'identifier et de quantifier la protéine et de définir à quelle température le produit destiné à l'alimentation a été chauffé ou quel traitement dénaturant physique, chimique ou enzymatique a été appliqué et à quel degré.

Par exemple, le réactif de dosage conforme à l'invention dirigé contre une protéine-test telle que l'ovalbumine sous ses formes native, partiellement dénaturée ou totalement dénaturée, peut doser l'ovalbumine sous ces diverses formes.

L'ovalbumine peut être introduite dans n'importe quel aliment destiné à être transformé par une méthode physique, chimique ou enzymatique.

Les réactifs conformes à l'invention permettent par exemple de distinguer un aliment qui n'a pas été chauffé ou qui a été chauffé entre 40° et 200°C, en définissant avec précision la température de chauffage.

La présente invention a par conséquent également pour objet un kit d'identification et de quantification d'au moins une substance protéique et de reconnaissance de la nature et du degré du traitement dénaturant au quel elle est soumise, lequel kit est caractérisé en ce qu'il comprend au moins un réactif immunochimique tel que défini plus haut, éventuellement au moins une protéine-test propre à être dosée par le réactif susdit, ainsi que les moyens de révélation usuels pour l'identification de la présence de la substance protéique à tester et/ou de la protéine-test.

Selon un mode de réalisation avantageux du kit conforme à la présente invention, la protéine-test est présente sous différentes formes et notamment sous ses formes native, partiellement dénaturée et totalement dénaturée.

Selon un mode de réalisation particulier du kit conforme à la présente invention, la protéine-test est de l'ovalbumine.

Outre les dispositions qui précèdent, l'invention comprend encore d'autres dispositions, qui ressortiront de la description qui va suivre.

L'invention sera mieux comprise à l'aide du complément de description qui va suivre, qui se réfère aux exemples de préparation et d'utilisation de réactifs conformes à l'invention.

Il doit être bien entendu, toutefois, que ces exemples sont donnés uniquement à titre d'illustration de l'objet de l'invention, dont ils ne constituent en aucune manière une limitation.

EXEMPLE 1 : Caractérisation d'anticorps polyclonaux vis-à-vis de l'ovalbumine native ou chauffée entre 50° et 130°C.

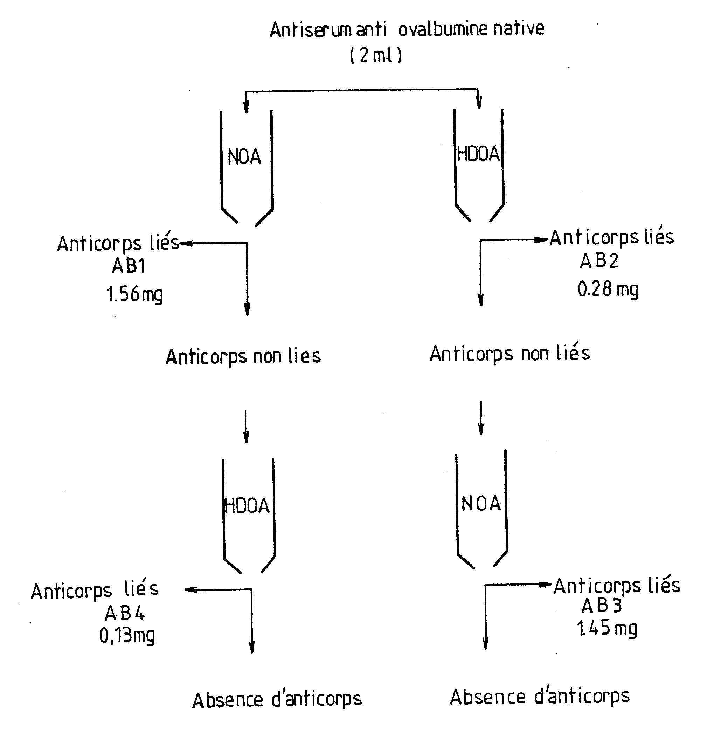

Ces anticorps proviennent de mammifères ou d'oiseaux tels que lapins, souris, chèvres, poulets, etc... immunisés soit contre de l'ovalbumine native, soit contre de l'ovalbumine dénaturée à différentes températures. On traite chacun des antiserums obtenus par chromatographie d'affinité, conformément à la figure 1 annexée, pour obtenir quatre sous-populations d'anticorps.

Par exemple, on obtient à partir d'un serum d'un lapin immunisé contre l'ovalbumine native, les sous-populations d'anticorps $NAC_1$, $NAC_2$, $NAC_3$, $NAC_4$ et s'il a été immunisé contre de l'ovalbumine dénaturée chauffée à 100 °C, les sous-populations d'anticorps $DAC_1$, $DAC_2$, $DAC_3$, $DAC_4$.

a) Caractérisation de ces sous-populations par immunocapture d'ovalbumine native ou dénaturée marquée par de l'iode radioactif.

Les anticorps purifiés par chromatographie d'affinité ont été ajustés à 1 µg/ml puis placés à raison de 100 µl par puits dans des plaques de 96 puits pendant 24h à 4°C. Après de nombreux lavages, on a ajouté des solutions soit d'ovalbumine native iodomarquée, soit d'ovalbumine chauffée à 100°C iodomarquée et incubée 3 heures à 37°C. Après lavage et découpage des puits, on a enregistré la radioactivité retenue dans chaque puits : connaissant la radioactivité spécifique de l'ovalbumine native et de l'ovalbumine dénaturée respectivement, on peut en déduire la quantité d'ovalbumine retenue. Par exemple, NAC$_1$ retient à la fois les deux antigènes mais 7 fois plus d'ovalbumine dénaturée que d'ovalbumine native, NAC$_3$ retient 2 fois plus d'ovalbumine dénaturée que d'ovalbumine native mais NAC$_4$ et surtout DAC$_4$ retiennent 20 à 100 fois plus d'ovalbumine dénaturée que d'ovalbumine native, comme le montre le Tableau I ci-dessous.

### TABLEAU I

Caractérisation des différents anticorps
polyclonaux par immunocapture.

|        | OAN | OAD |
|--------|-----|-----|
| OAN    | 3   | NF  |
| NAC$_1$ | 3,5 | 22  |
| NAC$_2$ | 1,5 | 37  |
| NAC$_3$ | 2,5 | 5   |
| NAC$_4$ | 0,1 | 1,9 |
| OAD    | NF  | 38  |
| DAC$_1$ | 1   | 34  |
| DAC$_2$ | 1   | 53  |
| DAC$_3$ | 0,8 | 11  |
| DAC$_4$ | 0,1 | 10  |

Résultats exprimés en nanogrammes
d'antigène fixé par la cupule en plaçant sur les
anticorps une solution d'antigène ou natif ou
chaffé à 100°C ajustée à 1µg/ml.

NF : non fait

L'immunocapture de l'antigène est proportionnelle à la quantité d'anticorps utilisée et fixée dans les puits : par exemple en utilisant 200 ng/ml d'anticorps DAC$_1$, au lieu de 1 µg/ml on ne retient pas du tout d'ovalbumine native et pourtant on retient plus de 5 ng d'ovalbumine dénaturée.

On peut affiner la technique en utilisant des AC$_1$, AC$_3$, AC$_4$ provenant de serums d'animaux immunisés contre l'ovalbumine chauffée à différentes températures.

b) Caractérisation des serums et des sous-populations d'anticorps polyclonaux par leur capacité d'identifier à quelle température l'ovalbumine a été chauffée.

En utilisant différents anticorps polyclonaux pour l'immunocapture et différents anticorps polyclonaux ou monoclonaux pour la révélation dans un test ELISA, on peut reconnaitre que l'ovalbumine a été chauffée à 65°C, à 85°C ou à 100 °C comme le montre le Tableau II ci-après.

## TABLEAU II

ANALYSE EN ELISA-SANDWICH (CAPTURE PAR ANTICORPS POLYCLONAUX DE LAPIN ET REVELATION PAR ANTICORPS POLYCLONAUX DE SOURIS OU MONOCLONAUX DE SOURIS) DE LA TEMPERATURE DE CHAUFFAGE DE L'OVALBUMINE.

| | NOA | 50°C | 65°C | 85°C | 200°C | 130°C |
|---|---|---|---|---|---|---|
| R$\alpha$OAN + MAb13 | 1000* | 500 | 500 | 0 | 0 | 0 |
| R$\alpha$OAN + MAb12 | 1000 | 1000 | 1000 | 0 | 0 | 0 |
| RAC$_1$D + MAb23 | 0 | 0 | 0 | 0 | 1000 | 1000 |
| RAC$_1$N + MAb23 | 0 | 0 | 0 | 0 | 900 | 900 |
| RAC$_3$N + MAb13 | 1000 | 1000 | 1000 | 0 | 1000 | 1000 |
| RAC$_3$D + MAb13 | 0 | 0 | 0 | 0 | 500 | 500 |
| R$\alpha$OAD + MAb13 | 0 | 0 | 0 | 500 | 0 | 0 |
| R$\alpha$OAD + M$\alpha$OAD | 0 | 0 | 0 | 0 | 900 | 900 |
| R$\alpha$OAN + M$\alpha$OAN | 1000 | 1000 | 1000 | 700 | 0 | 0 |

R$\alpha$OAD Anticorps de lapins anti-ovalbumine dénaturée purifiés par précipitation à $SO_4(NH_4)_2$

R$\alpha$OAN Anticorps de lapins anti-ovalbumine native purifiés par précipitation à $SO_4(NH_4)_2$

RAC$_1$N RAC$_3$N RAC$_1$D RAC$_3$D Anticorps purifiés par chromatographie d'affinité

M$\alpha$OAD Anticorps de souris anti-ovalbumine dénaturée purifiés par précipitation à $SO_4(NH_4)_2$

M$\alpha$OAN Anticorps de souris anti-ovalbumine native purifiés par précipitation à $SO_4(NH_4)_2$

* Les chiffres représentent les densités optiques obtenues 15 mn après l'addition du substrat

EP 0 343 064 A1

On peut traiter de la même manière un sérum provenant d'un animal immunisé contre de l'ovalbumine dénaturée à 135°C ; chacun des anticorps purifiés ainsi possède des propriétés de reconnaissance de l'ovalbumine différentes. L'ovalbumine peut être ajoutée par exemple à des conserves de viandes, de légumes, de champignons ou de produits lactés destinées à être préparées ou stérilisées par traitement à la chaleur de 40° à 200°C.

On peut également préparer des anticorps polyclonaux contre les protéines constituants de l'aliment telles que pour la viande de boeuf, une protéine thermostable, notamment l'inhibiteur de cystéine-protéinase ou une protéine thermosensible telle que l'inhibiteur de sérine-protéinase. Dans le cas du lait on peut choisir par exemple la β-lactoglobuline thermorésistante et la K caséine thermosensible. Pour les végétaux, différentes enzymes thermosensibles ou des inhibiteurs de protéases thermorésistants : dans chaque cas, la modification de la carte épitopique permet de reconnaître le traitement et en particulier le degré de chauffage auquel l'aliment a été soumis.

Les techniques d'identification de la spécificité de ces anticorps sont classiques et réalisées par différentes méthodes ELISA (Engvall E. et Perlmann P. J. Immunol. 1972, 109, 129) directe, indirecte ou par compétition.

La combinaison RαOAN et MAb13 donne une réaction positive pour l'ovalbumine native ou chauffée à 50°C ou à 65°C mais négative pour l'ovalbumine chauffée à 85°C et à 100°C. La combinaison RαOAD et MAb13 ne donne de réaction positive que lorsque l'ovalbumine a été chauffée à 85°C. La combinaison RAC3N et MAb13 donne une réaction négative seulement pour l'ovalbumine à 85°C mais inversement la combinaison RαOAD et MAb13 donne des réactions négatives dans tous les cas sauf pour l'ovalbumine chauffée à 85°C.

EXEMPLE 2 : Caractérisation des anticorps monoclonaux vis-à-vis de l'ovalbumine native ou chauffée entre 50°C et 130°C

Les hybridomes ont été obtenus chez des souris immunisées soit contre de l'ovalbumine native, soit contre de l'ovalbumine dénaturée à différentes températures. Après immunisation la rate de la souris est dissociée et la fusion cellulaire est pratiquée avec un plasmocytome de souris comme l'ont décrit G. KOHLER et C. MILSTEIN (Nature 1975). A partir de 90 hybridomes reconnus spécifiques, des clones cellulaires produisant des anticorps spécifiques ont été obtenus dont 23 seront décrits, dans ce qui va suivre, pour leurs propriétés particulières.

Dans le Tableau III ci-dessous sont résumées certaines propriétés de ces anticorps monoclonaux - les classes et sous-classes ont été identifiées par des anticorps spécifiques par une méthode ELISA. La spécificité des anticorps dans la reconnaissance de l'antigène a été validée à la fois par ELISA direct et par ELISA sandwich. Dans cette capacité de reconnaissance, on trouve des anticorps très spécifiques de l'ovalbumine native (11, 12, 13, 15, 21), d'autres très spécifiques de l'ovalbumine chauffée à 100°C (1, 2, 3, 5, 16) et beaucoup reconnaissent les deux formes mais pas toujours avec la même technique (4, 6, 9, 14, 19, 22, 23). Ceci est confirmé par l'étude de l'immunocapture de l'ovalbumine native ou de l'ovalbumine chauffée à 100°C : certains sont très spécifiques de l'ovalbumine native (15, 17, 21), d'autres de l'ovalbumine dénaturée (1, 2, 5, 7, 10), la plupart capturent les deux formes (la capture est dose-dépendante).

EP 0 343 064 A1

TABLEAU III

Caractérisation des anticorps monoclonaux anti-ovalbumine.

| N | Classe ss-classe | Spécificité (Détection) | | | | Immunocapture | |
|---|---|---|---|---|---|---|---|
| | | A | B | C | D | E | F |
| 1 | μ | | + | | | | |
| 2 | μ | | + | | | 0 | 9 |
| 3 | μ | | + | | | | 19 |
| 4 | 1 | | + | + | + | 0 | 21 |
| 5 | μ | | + | | | 0 | 27 |
| 6 | μ + 2b | | + + | + | + | 0,22 | 19 |
| 7 | μ | | + | | | 0 | 19 |
| 8 | μ | | + | | | 0,08 | 11 |
| 9 | μ | | + | + | + | 0 | 8 |
| 10 | 1+μ | | + | | | 0 | 7 |
| 11 | 1 | | | + | | 5,5 | 3 |
| 12 | 1 | | | + | | 1,7 | 1 |
| 13 | 1 | | | + | | 3 | 3 |
| 14 | 1 | | + + | + | + | 0,15 | 2,5 |
| 15 | 1 | | | + | | 12 | 0,6 |
| 16 | 1 | | + | | | 0,08 | 52 |
| 17 | 2b | | | + | | 0,11 | 0 |
| 18 | 1 | | | + | | 0 | 0 |
| 19 | 1 | + | + | + + | + | 0,17 | 4 |
| 20 | 1 | | | + | | 0 | 0 |
| 21 | 1 | | | + | | 1,18 | 0 |
| 22 | 1 | | | + | + | 1,75 | 0 |
| 23 | 1 | | + + | + | + | 0,40 | 4,3 |

A mesure par ELISA direct (NOA)

B " " " (DOA, chauffée à 100°C)

C mesure par ELISA sandwich (NOA)

D " " " (DOA, chauffée à 100°C)

E exprimée en nanogrammes de NOA marquée retenue

F " " " DOA " "

Le Tableau IV ci-après montre comment certains anticorps monoclonaux utilisés pour l'immunocapture permettent, en révélant la présence ou l'absence d'antigène par divers anticorps polyclonaux, d'identifier soit l'ovalbumine native, soit l'ovalbumine chauffée à 50°C, 65°C, 85°C, 100°C ou 130°C.

8

TABLEAU IV

ANALYSE EN ELISA-SANDWICH DE LA TEMPERATURE DE CHAUFFAGE DE L'OVALBUMINE (CAPTURE PAR DES ANTICORPS MONOCLONAUX ET REVELATION PAR ANTICORPS POLYCLONAUX DE LAPIN)

| | NOA | 50°C | 65°C | 85°C | 100°C | 130°C |
|---|---|---|---|---|---|---|
| 7 + NAC$_2$ | 0 | 0 | 0 | 1000 | 1000 | Non fait |
| 9 + NAC$_2$ | 0 | 0 | 0 | 800 | 1000 | Non fait |
| 13 + NAC$_2$ | 1000 | 1000 | 1000 | 300 | 400 | Non fait |
| 14 + DAC$_3$ | 0 | 0 | 0 | 0 | 700 | 700 |
| 15 + NAC$_2$ | 1000 | 1000 | 1000 | 0 | 0 | Non fait |
| 16 + NAC$_2$ | 700 | 0 | 0 | 1000 | 1000 | Non fait |
| 11 + NAC$_2$ | 1000 | 1000 | 1000 | 500 | 0 | Non fait |
| 20 + NAC$_3$ | 700 | 700 | 700 | 0 | 0 | Non fait |
| 21 + NAC$_2$ | 1000 | 1000 | 1000 | 0 | 0 | Non fait |
| 23 + NAC$_2$ | 0 | 0 | 300 | 600 | 1000 | Non fait |

EP 0 343 064 A1

De la même manière, on peut, pour un produit alimentaire, préparer des anticorps monoclonaux contre un ou plusieurs constituants du produit, en choisissant de préférence une protéine thermosensible et une protéine thermorésistante (pour la viande de boeuf, par exemple un inhibiteur de cystéine-protéinase est thermostable alors que l'inhibiteur de sérine-protéinase est thermosensible).

EXEMPLE 3 : Application de la méthode d'identification à des conserves additionnées de blanc d'oeuf.

On pouvait craindre que les épitopes spécifiques de la conformation des protéines dépendante de la température soient dénaturés en présence d'un excès de glycoprotéines, de polysaccharides ou de lipides. Les Inventeurs ont observé qu'il n'en était rien en additionnant du blanc d'oeuf à des conserves de champignons chauffées aux différentes températures étudiées, le problème majeur pour les chauffages à 85°C, 100°C ou 130°C étant la solubilisation que l'on résoud à l'aide des solubilisants classiques. L'on pratique souvent une centrifugation pour éliminer les produits non solubilisés.

Dans le présent exemple, on utilise soit le jus, soit les champignons broyés : pour le cas où le chauffage n'a pas dépassé 65°C on extrait ou on dilue par du tampon phosphate ; pour les températures de 85°C, 100°C et 130°C, les produits sont traités pendant 10 minutes à 48h par de la soude ajustée à 0,1 Molaire. L'on ajuste dans ce cas la concentration de protéines totales entre 30 ng/ml et 30 μg/ml.

Le Tableau V qui va suivre montre que l'on peut identifier dans la conserve de champignons la présence d'ovalbumine et détecter les températures de chauffage de 65°C, 85°C, 100°C et 130°C de façon différente à l'aide d'anticorps polyclonaux. Il en est de même avec les anticorps monoclonaux (cf. Tableau VI ci-après) utilisés pour l'immunocapture. Certains anticorps polyclonaux et monoclonaux sont capables de reconnaître l'ovalbumine après traitement par thermoextrusion.

EP 0 343 064 A1

TABLEAU V :    IDENTIFICATION DE LA TEMPERATURE DE CHAUFFAGE D'UNE CONSERVE PAR ANTICORPS POLYCLONAUX (IMMUNOCAPTURE PAR ANTICORPS POLYCLONAUX DE LAPIN ET REVELATION PAR ANTICORPS POLYCLONAUX DE SOURIS OU PAR ANTICORPS MONOCLONAUX DE SOURIS) [1]

|  | Non chauffée | 50°C | 65°C | 85°C | 100°C | 130°C |
|---|---|---|---|---|---|---|
| RNAC$_2$ + MDOA (a) | 500 | 500 | 700 | 0 | 0 | 0 |
| RNAC$_3$ + MDOA (a) | 600 | 600 | 600 | 0 | 0 | 0 |
| RDAC$_1$ + MDOA (b) | 0 | 0 | 0 | 600 | 700 | 0 |
| RDAC$_4$ + MDOA (b) | 0 | 0 | 0 | 700 | 700 | 0 |
| RNAC$_1$ + MDOA (c) | 0 | 0 | 0 | 0 | 300 | 300 |
| RDAC$_2$ + MDOA (c) | 0 | 0 | 0 | 0 | 0 | 400 |
| RαOAN + 13 | 900 | 500 | 500 | 500 | 0 | 0 |
| RαOAN + 12 | 1000 | 600 | 600 | 600 | 0 | 0 |
| RαOAN + 17 | 600 | 300 | 300 | 0 | 0 | 0 |
| RαOAN + MαOAN | 1000 | 800 | 800 | 800 | 0 | 0 |
| RαOAD + MαOAD | 0 | 0 | 0 | 0 | 700 | Non fait |

[1] Les extraits de champignons ont été ajustés soit à 30 ng (a)
soit à 100 ng (b)
soit à 25 µg (c)

TABLEAU VI

IDENTIFICATION DE LA TEMPERATURE A LAQUELLE UNE CONSERVE A ETE CHAUFFEE, PAR IMMUNOCAPTURE AVEC DES ANTICORPS MONOCLONAUX

| | NOA | 50°C | 65°C | 85°C | 100°C | | 130°C |
|---|---|---|---|---|---|---|---|
| 3 + NAC2 | 0 | 0 | 0 | 1400[1] | 1600[1] | | 1000[1] |
| 4 + NAC2 | 0 | 0 | 0 | 0 | 1100 | Non fait | |
| 9 + NAC2 | 0 | 0 | 0 | 0 | 1000 | | 0 |
| 13 + NAC2 | 1700 | 1700 | 1700 | 800 | 300 | | 0 |
| 15 + NAC2 | 1800 | 1800 | 1800 | 0 | 0 | | 0 |
| 21 + NAC2 | 900 | 900 | 900 | 0 | 0 | | 0 |
| 23 + NAC2 | 0 | 0 | 0 | 0 | 900 | | 1400[1] |

1 La solution de protéines est ajustée ici à 100 ng/ml sauf les chiffres 1 où l'on a mis 100 µg/ml

EP 0 343 064 A1

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1°) Réactif immunochimique d'identification et de quantification d'au moins une substance protéique et de reconnaissance de la nature et du degré du traitement dénaturant auquel elle est soumise, lequel réactif est caractérisé en ce qu'il est constitué par des anticorps dirigés contre ladite ou lesdites substance(s) protéique(s) et présentant différentes spécificités qui correspondent chacune à un état de ladite ou desdites substance(s) protéique(s).

2°) Réactif selon la revendication 1, caractérisé en ce qu'il est constitué par des anticorps dirigés contre ladite (lesdites) substance(s) protéique(s) à l'état natif et/ou contre ladite (lesdites) substance(s) protéique(s) dénaturée(s) à différentes températures et/ou contre ladite (lesdites) substance(s) protéique(s) dénaturée(s) par un traitement approprié tel, notamment, qu'un traitement physique, d'extrusion en particulier, un traitement chimique ou un traitement enzymatique.

3°) Réactif selon l'une quelconque des revendications 1 et 2, caractérisé en ce qu'il est dirigé contre au moins une substance protéique qui fait -ou font- partie des constituants du produit alimentaire analysé.

4°) Réactif selon l'une quelconque des revendications 1 et 2, caractérisé en ce qu'il est dirigé contre au moins une substance protéique-test ajoutée au produit alimentaire à analyser, préalablement au traitement, thermique, physique, chimique, enzymatique ou autre, auquel elle doit être soumise.

5°) Réactif selon l'une quelconque des revendications 1 à 4, caractérisé en ce qu'il est constitué par des anticorps polyclonaux.

6°) Réactif selon l'une quelconque des revendications 1 à 4, caractérisé en ce qu'il est constitué par des anticorps monoclonaux.

7°) Procédé de production d'un réactif immunochimique d'identification et de quantification d'une substance protéique, constitué par des anticorps dirigés contre ladite (ou lesdites) substances(s) protéique(s) et présentant différentes spécificités correspondant chacune à un état de ladite ou desdites substance(s) protéique(s), lequel procédé est caractérisé en ce que des mammifères ou des oiseaux appropriés sont immunisés par au moins une substance protéique présentant différents états, et notamment différents états de dénaturation, en ce que les anticorps polyclonaux ou monoclonaux obtenus à la suite de cette immunisation et dirigés contre les différents états de ladite substance protéique, sont recueillis pour servir de réactifs immunochimiques aptes à détecter et à quantifier les différents états de dénaturation de ladite substance dans un produit alimentaire.

8°) Procédé selon la revendication 7, caractérisé en ce que la substance protéique utilisée dans plusieurs de ses états notamment de dénaturation, pour immuniser des mammifères ou des oiseaux appropriés, est identique à une substance protéique qui entre dans la composition du produit alimentaire à analyser.

9°) Procédé selon la revendication 7, caractérisé en ce que la substance protéique utilisée dans plusieurs de ses états, notamment de dénaturation, pour immuniser des mammifères ou des oiseaux appropriés, est une substance protéique identique à une protéine ajoutée au produit alimentaire à analyser, en tant que protéine-test connue pour son aptitude à entraîner l'apparition de différents déterminants antigéniques sensibles aux anticorps susdits.

10°) Procédé selon la revendication 7, appliqué à la production d'anticorps polyclonaux, caractérisé en ce que le(s) serum(s) recueilli(s) après immunisation de mammifères ou d'oiseaux appropriés par au moins une protéine constituant du produit alimentaire ou par au moins une protéine-test native ou dans différents états de dénaturation, et contenant des anticorps polyclonaux est/sont traité(s) par un agent de précipitation propre à provoquer la précipitation des immunoglobulines pour éliminer des protéines sériques susceptible de diminuer la sensibilité de la méthode.

11°) Procédé selon la revendication 7, appliqué à la production d'anticorps polyclonaux, caractérisé en ce que le(s) serum(s) recueilli(s) après immunisation de mammifères ou d'oiseaux appropriés par au moins une protéine-constituant du produit alimentaire ou par au moins une protéine-test, dans différents états de dénaturation et contenant des anticorps polyclonaux, est/sont traité(s) sur une colonne de chromatographie d'affinité pour séparer les différentes spécificités contenues dans lesdits sérums anti-substance protéique en vue de leur utilisation comme réactifs aptes à reconnaître chacune l'un des différents états de ladite substance protéique, dans le produit alimentaire à analyser.

12°) Procédé d'identification et de quantification d'au moins une substance protéique et de reconnaissance de la nature et du degré du traitement dénaturant auquel elle est soumise, caractérisé en qu'un réactif immunochimique, tel qu'un anticorps, apte à détecter différents états , notamment de dénaturation, d'une substance protéique présente dans un produit à analyser, notamment dans un produit alimentaire, est mis en contact avec le produit à analyser, puis le complexe antigène-anticorps formé est détecté et quantifié par une technique immunoenzymatique appropriée.

13°) Procédé selon la revendication 12, caractérisé en ce que le produit à analyser est soumis préalablement à la détection de l'antigène (protéique) qu'il contient, par une technique immunoenzymatique, à un processus d'extraction propre à solubiliser les protéines prsentes dans ledit produit.

14°) Kit d'identification et de quantification d'au moins une substance protéique et de reconnaissance de la nature et du degré du traitement dénaturant auquel elle est soumise, lequel kit est caractérisé en ce qu'il comprend au moins un réactif immunochimique selon l'une quelconque des revendications 1 à 6, éventuellement au moins une protéine-test propre à être dosée par le réactif susdit, ainsi que les moyens de révélation usuels pour l'identification de la présence de la substance protéique à tester et/ou de la protéine-test.

15°) Kit selon la revendication 14, caractérisé en ce que la protéine-test est présente sous différentes formes et notamment sous ses formes native, partiellement dénaturée et totalement dénaturée.

Antiserum anti ovalbumine native
(2ml)

NOA

HDOA

Anticorps liés
AB1
1.56 mg

Anticorps liés
AB2
0.28 mg

Anticorps non lies

Anticorps non liés

HDOA

NOA

Anticorps liés
AB4
0,13 mg

Anticorps liés
AB3
1.45 mg

Absence d'anticorps

Absence d'anticorps

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 105, no. 11, 15 septembre 1986, page 521, résumé no. 96138m, Columbus, Ohio, US; F.W. JANSSEN et al.: "Detection of soya proteins in heated meat products by "blotting" and "dot blot"", & Z. LEBENSM.-UNTERS. FORSCH. 1986, 182(6), 479-83 * En entier * | 1-15 | G 01 N 33/68 G 01 N 33/02 |
| Y | CHEMICAL ABSTRACTS, vol. 106, no. 13, 30 mars 1987, page 543, résumé no. 100990y, Columbus, Ohio, US; P.J. KILSHAW et al.: "Studies on the specificity of antibodies to ovalbumin in normal human serum: technical considerations in the use of ELISA methods", & CLIN. EXP. IMMUNOL. 1986, 66(2), 481-9 * En entier * | 1-15 | |
| D,Y | JOURNAL OF FOOD SCIENCE, vol. 53, no. 1, 1988, pages 222-225, Chicago, US; C. BRETON et al.: "Immunochemical properties of native and heat denatured ovalbumin" * En entier * | 1-15 | TECHNICAL FIELDS SEARCHED (Int. Cl.4) G 01 N |
| Y | JOURNAL OF FOOD SCIENCE, vol. 53, no. 1, 1988, pages 226-230, Chicago, US; C. BRETON et al.: "Immunochemical identification and quantification of ovalbumin additive in canned mushrooms" * En entier *        -/- | 1-15 | |

The present search report has been drawn up for all claims

| Place of search | Date of completion of the search | Examiner |
|---|---|---|
| THE HAGUE | 27-06-1989 | GRIFFITH G. |

European Patent
Office

EUROPEAN SEARCH REPORT

Application Number

EP 89 40 1357

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | JOURNAL OF THE SCIENCE OF FOOD AND AGRICULTURE, vol. 39, 1987, pages 179-184, Society of Chemical Industry, Oxford, GB; E.K. KANG'ETHE et al.: "Thermostable muscle antigens suitable for use in enzyme immunoassays for identification of meat from various species" * En entier * | 1-15 | |
| Y | CHEMICAL ABSTRACTS, vol. 107, no. 5, 3 août 1987, page 558, résumé no. 38147u, Columbus, Ohio, US; F.W. JANSSEN et al.: "Detection of wheat gluten, whey protein, casein ovalbumin, and soy protein in heated meat products by electrophoresis, blotting, and immunoperoxidase staining", & J. AGRIC. FOOD CHEM. 1987, 35(4), 563-7 * En entier * | 1-15 | |
| D,A | AGRICULTURAL AND BIOLOGICAL CHEMISTRY, vol. 49, no. 2, 1985, pages 423-427, Tokyo, JP; Y. KATO et al.: "Alteration of ovalbumin immunogenic activity by glycosylation through maillard reaction" | | TECHNICAL FIELDS SEARCHED (Int. Cl.4) |
| A | CHEMICAL ABSTRACTS, vol. 98, no. 9, 28 février 1983, page 501, résumé no. 70566m, Columbus, Ohio, US; J.W. SISSONS et al.: "Ethanol denaturation of soya bean protein antigens", & J. SCI. FOOD AGRIC. 1982, 33(8), 706-10 | | |

The present search report has been drawn up for all claims

| Place of search | Date of completion of the search | Examiner |
|---|---|---|
| THE HAGUE | 27-06-1989 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
....................................
& : member of the same patent family, corresponding document

EPO FORM 1503 03.82 (P0401)